# EUROPEAN PATENT SPECIFICATION

(11) **EP 1 440 661 B1**
(45) Date of publication and mention of the grant of the patent: **15.07.2009**
(21) Application number: 04100076.1
(22) Date of filing: 12.01.2004
(51) Int. Cl.: A61B 17/00

(54) **Closure device**
Verschlussvorrichtung
Dispositif d'occlusion

(30) Priority: 14.01.2003 US 439800
(43) Date of publication of application: 28.07.2004
(73) Proprietor: RADI MEDICAL SYSTEMS AB, 754 50 Uppsala (SE)
(72) Inventor: Egnelöv, Per, 754 40, Uppsala (SE); Preinitz, Fredrik, 753 50, Uppsala (SE)
(74) Representative: Lindgren, Anders

(56) References cited:
- EP-A- 1 266 626
- WO-A-00/78226
- US-A- 5 662 681
- US-A1- 2001 003 158
- US-B1- 6 325 789

## Description

### FIELD OF THE INVENTION

The present invention relates generally to a closure device for sealing a percutaneous puncture in the wall of a blood vessel, and more particularly to a closure device and a method by which an inner seal is deployed inside a vessel and a locking member is secured outside the vessel, such that bleeding from the percutaneous puncture is prevented.

### BACKGROUND OF THE INVENTION

A system for sealing a percutaneous puncture in a blood vessel can comprise an inner seal which is adapted to be positioned against an inner surface of the vessel wall, and a locking member which is connected to the inner seal by, for example, a filament or a suture, and which is adapted to be positioned against an outer surface of the vessel wall such that the percutaneous puncture is sealed there between. During the phase of insertion, the inner seal is folded inside an introducer tube which is resting in the puncture to provide access to the interior of the blood vessel. Deployment of the inner seal inside the vessel takes place by pushing the inner seal through the tube, out from the distal end opening of the introducer. To ensure proper unfolding of the inner seal, the inner seal has to be deployed some distance away from the puncture hole in the vessel wall before the inner seal is positioned to be securely seated against the inner surface of the vessel wall. When the inner seal has been deployed inside the blood vessel, the introducer is retracted from the puncture to rest with its distal end outside the vessel, in close proximity to the puncture. When, during said retraction, the inner seal has been positioned against the vessel wall to cover the puncture, the locking member is pushed forward through the introducer tube until the locking member is tamped in contact with the outer surface of the vessel wall. To effectuate the different actions described above, inserter tools have been proposed which also accommodate the inner seal and the locking member before the sealing procedure.

The devices and procedures for sealing a percutaneous puncture in a blood vessel may be improved with respect to drawbacks connected with the prior art systems:

For example, existing systems unconditionally allow tamping of the locking member and provide no verification that the distal end of the inserter is retracted from the puncture before tamping, leading to a possibility of the locking member unintentionally being positioned inside the vessel in case of an incorrect manoeuvre.

Another drawback in existing systems is that two hands typically are required to handle existing tools for deployment of the inner seal, seating the inner seal against the vessel wall, and for tamping of the locking member.

Wound closure devices and methods are previously known. US 6,325,789 B1, e.g., discloses a device by which hemostatic material is inserted through a tissue channel to be deployed against the exterior of a blood vessel for sealing a puncture through the wall of the vessel. This device comprises basically a sheath and a plug pusher or piston. The sheath is inserted through body tissue until its distal end abuts the exterior of the vessel wall, whereupon the hemostatic material plug is advanced through the sheath by use of the plug pusher until the plug abuts the vessel wall, on the outside thereof. The sheath and plug are oversized with respect to the puncture size so that the hemostatic material plug overlaps the puncture on all sides thereof.

A wound closure device is previously known also from US 5,662,681. This closure device is arranged to be inserted into a blood vessel by means of an introducing instrument, via a puncture through the vessel wall. This device basically comprises an anchor adapted to be positioned inside the vessel, a collagen plug adapted to be positioned on the outside of the vessel, a locking disk, and a flexible filament connecting the previous members. The filament is guided through the members in a loop configuration, by which the locking disk effects a compression of the collagen plug towards the vessel exterior in result of a pulling force applied to the filament.

A tool which is structured for mating two plug members of a sealing device in a wound closing procedure is known from WO 00/78226 A1. This tool comprises a mechanism concealed in a housing. A sliding lever is accessible from outside the housing. The lever engages the mechanism and is operable for moving the mechanism in a distal direction upon insertion and deployment of an inner plug which is to be positioned inside a vessel. To this purpose, the lever is moved by an operator towards an end position formed on the housing. A filament that connects the inner plug with the lever engages the mechanism through a filament loop.

The mechanism in the tool of WO 00/78226 Al is arranged for converting a tensioning movement, by which the filament is tensioned in result of moving the tool away from the wound, into a pushing movement towards the wound whereby an outer plug is positioned outside the vessel, in a tamping operation, so as to be connected with the inner plug in a tamping operation. In the known tool the tamping operation is a direct result of tensioning of the filament. In the tool of WO 00/78226 Al, the lever is thus actuated once by the operator who moves the lever into a forward end position in the housing in order to effect insertion of the inner plug. The lever is however not repeatedly actuated also for deposition of the outer plug, as is the case of the present invention. In contrast to the claimed invention, the lever of the prior tool is stationary in the housing in the tamping mode, and effects then merely a detachable arrest of the filament's proximal end for tensioning purposes.

### SUMMARY OF THE INVENTION

An overall object of the present invention is to provide a closure device and method by which the proper closure and ease of handling are both enhanced upon sealing of a percutaneous puncture in the wall of a blood vessel after a medical treatment or surgery.

In one aspect, the present invention aims to provide a closure device by which is eliminated the possibility of the locking member being unintentionally positioned inside the blood vessel.

In another aspect, the present invention aims to provide a closure device by which it is ensured that the inner seal is seated against the vessel wall before tamping of the locking member.

In a further aspect, the present invention aims to provide a closure device and method by which: deployment of an inner seal inside the vessel; seating the inner seal against the inner side of the vessel wall; and tamping of a locking member such that bleeding through the puncture is prevented, may all be performed in a one-hand operation.

These and other aims and aspects of the invention will be met by a closure device as defined in claim 1, and by the method as defined in claim 12. Advantageous embodiments are further specified in the subordinated claims.

Briefly, the present invention provides a closure device for sealing a percutaneous puncture in the wall of a blood vessel, comprising an insertion tool having an actuator which is operable in a first mode for deployment of an inner seal inside the vessel and operable in a second mode for tamping of a locking member outside the vessel, the actuator being arranged to be set into said second operable mode in response to a pulling force acting on a filament connecting the inner seal and the locking member.

Preferably, the actuator is controlled by an actuator mechanism that is adapted to disable the actuator until a pulling force acting on the filament causes the actuator to be reset into said second operable mode.

In accordance herewith, a method of sealing a percutaneous puncture through the wall of a blood vessel is disclosed wherein tamping of the locking member is enabled through the step of applying a pulling force to act on a filament connecting the inner seal and the locking member. In a preferred embodiment, tamping of the locking member is disabled until a pulling force is applied to act on the filament.

### SHORT DESCRIPTION OF THE DRAWINGS

The invention is more closely described below by reference to the drawings, diagrammatically illustrating the new insertion tool and method. In the drawings,
Fig. 1 illustrates a blood vessel in which a distal end of an introducer (prior art) is positioned;
Fig. 2 illustrates a guide rod being inserted through the introducer of fig. 1;
Fig. 3 illustrates the vessel in which only the guide rod is in place;
Fig. 4 illustrates a closure device according to the present invention, the closure device being positioned over the guide rod of fig. 3;
Fig. 5 illustrates how the guide rod is removed from the closure device and from the vessel;
Fig. 6 is a diagrammatic, cross-sectional view of internal passageways that guide the seal assembly and the guide rod, respectively, in a housing of the closure device;
Fig. 7 illustrates how an actuator is pushed into the housing for deployment of an inner seal in a first mode of operation;
Fig. 8 illustrates how the closure device is retracted until the inner seal is in contact with the vessel wall;
Fig. 9 illustrates how the actuator is pushed into the housing for tamping of a locking member in a second mode of operation;
Fig. 10 illustrates the closure device after completion of the sealing operation;
Fig. 11 is an exploded view showing the major components of one embodiment of the new closure device;
Fig. 12 is a partially broken away elevation view of a seal assembly and associated elements of the closure device;
Fig. 13a is a partially broken away elevation view of a slider, incorporated in the closure device;
Fig. 13b is a top view of the slider of fig. 13a;
Fig. 13c shows the proximal end of the slider;
Fig. 13d shows the distal end of the slider;
Fig. 14a is a longitudinal section, part of which is laterally displaced, through an actuator incorporated in the closure device;
Fig. 14b is a sectional top view of the actuator of fig. 14a;
Fig. 14c is an elevation view showing the distal end of the actuator;
Fig. 14d is a partially broken away sectional view showing the slider and the actuator in a first relative position, realizing a first mode of operation;
Fig. 14e is a partially broken away sectional view showing the slider and the actuator in a second relative position, realizing a second mode of operation;
Fig. 15a is a longitudinal section through a sleeve, incorporated in the closure device;
Fig. 15b is a sectional top view of the sleeve of fig. 15a;
Fig. 15c is an elevation view showing the proximal end of the sleeve.

### DETAILED DESCRIPTION

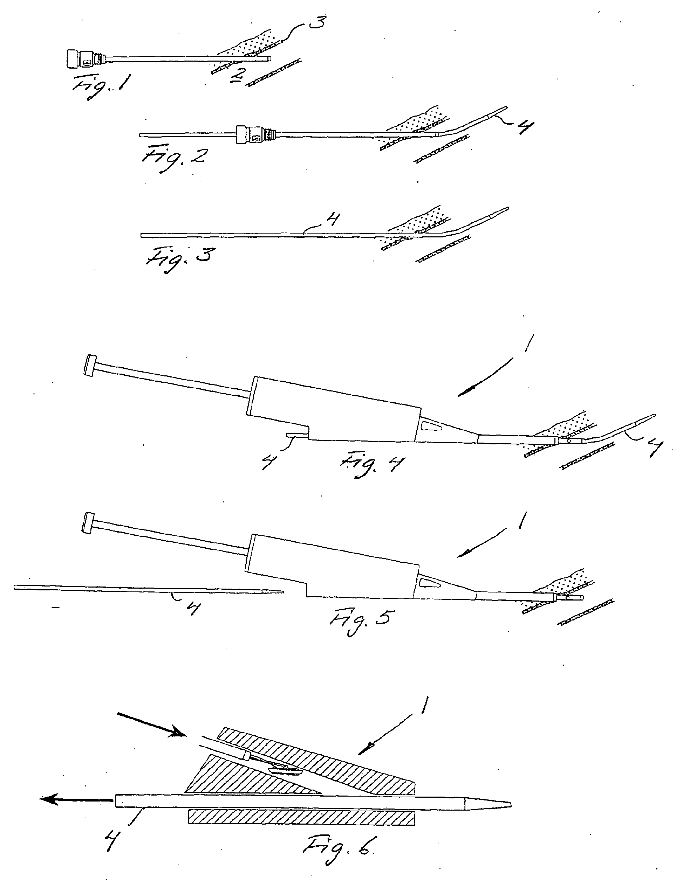

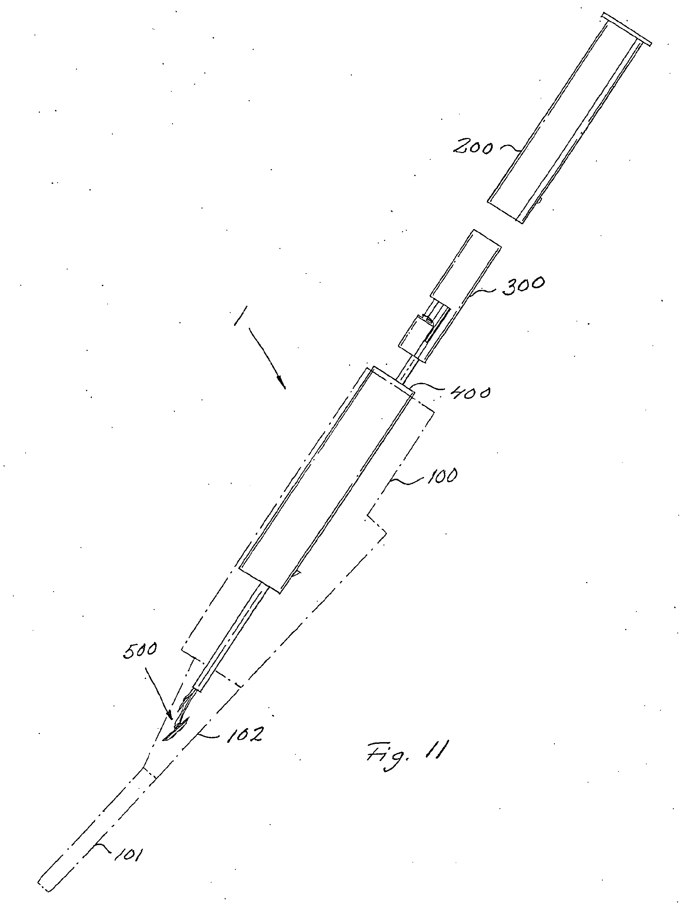

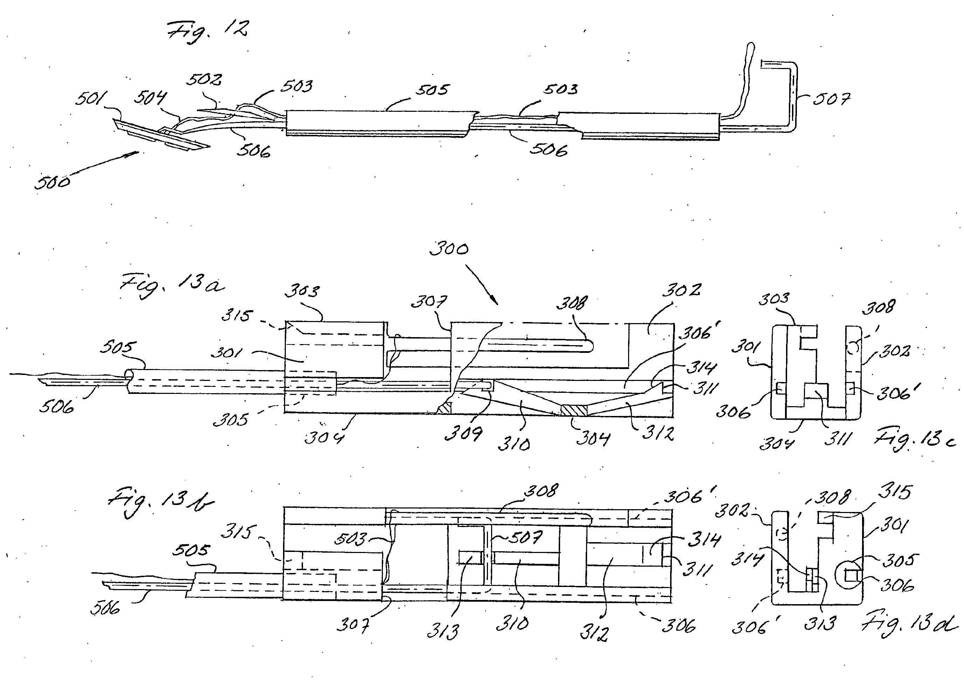

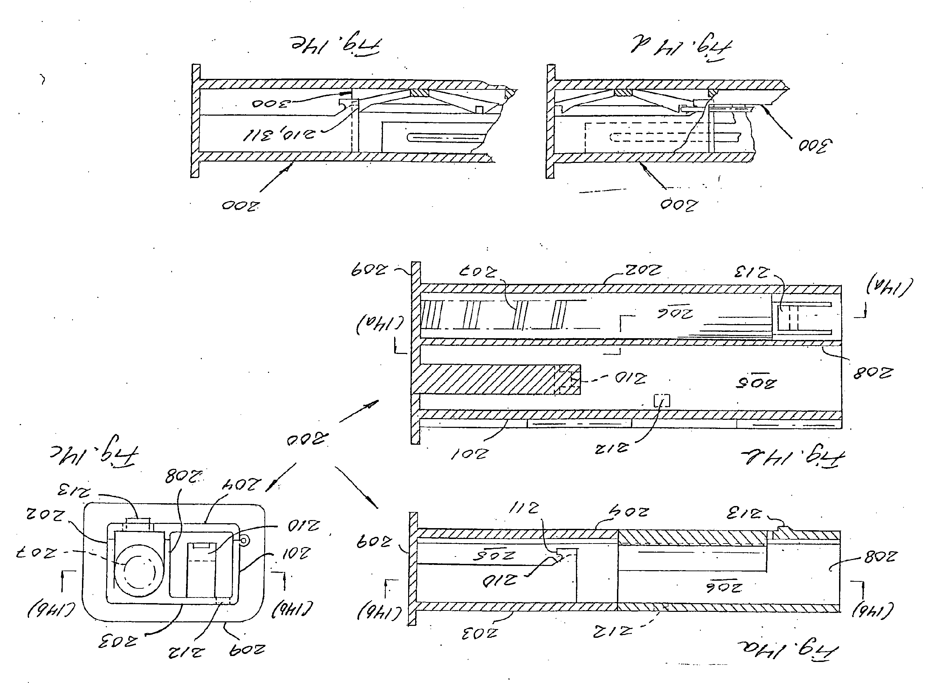

A closure device for sealing a percutaneous puncture in a blood vessel is generally referred to by reference numeral 1 in the drawings. In figs. 4-10, the closure device 1 is diagrammatically shown in different operative positions illustrating the procedural steps of the sealing operation. In figs. 11-15, the structure and operation of an actuator mechanism in the closure device 1 is illustrated and explained, by way of example. For reasons of visibility, figs. 12 and 13 are drawn on a larger scale with respect to the drawings of figs. 14-15.

### METHOD

Fig. 1 illustrates an introducer (prior art) whose distal end portion being introduced into a blood vessel 2 and whose proximal portion extends out from the skin of a patient. Presumably, a medical operation has been performed via the introducer, and the puncture hole through the wall 3 of the blood vessel is now to be closed.

In order to replace the introducer with a closure device according to the present invention, a guide rod 4 may be inserted through the introducer as is shown in fig. 2.

In fig. 3 the introducer has been removed, leaving only the guide rod in place. An advantage achieved by the use of a guide rod is that the diameter of the guide rod is larger than the diameter of a guide wire, e.g., which in the case of an artery results in less blood flowing from the artery, and a manual external compression may thereby be avoided.

Fig. 4 shows a closure device 1 according to the present invention, threaded over the guide rod. This is in contrast to insertion tools that are adapted to be connected to an existing introducer. The present closure device is therefore independent of the type, e.g. diameter or length, of the introducer that was previously inserted into the vessel.

When the correct positioning of the closure device 1 has been established, the guide rod is removed as is illustrated in figs. 5 and 6.

Fig. 6 shows, diagrammatically, a cross-section of a forward portion of the housing of the closure device 1. From the drawing it can be seen that a first passageway, in which an inner seal and a locking member are positioned, connects to a second passageway in which the guide rod moves. Here it should be noted that it is possible to let the first and second passageways switch places, such that the guide rod, which is flexible, is inserted through a passageway that connects to a straight passageway through which the inner seal is inserted. It is also conceivable to have two passageways that both are slightly bent. Two passageways can also connect to a straight passageway, which gives a configuration having the shape of the letter Y.

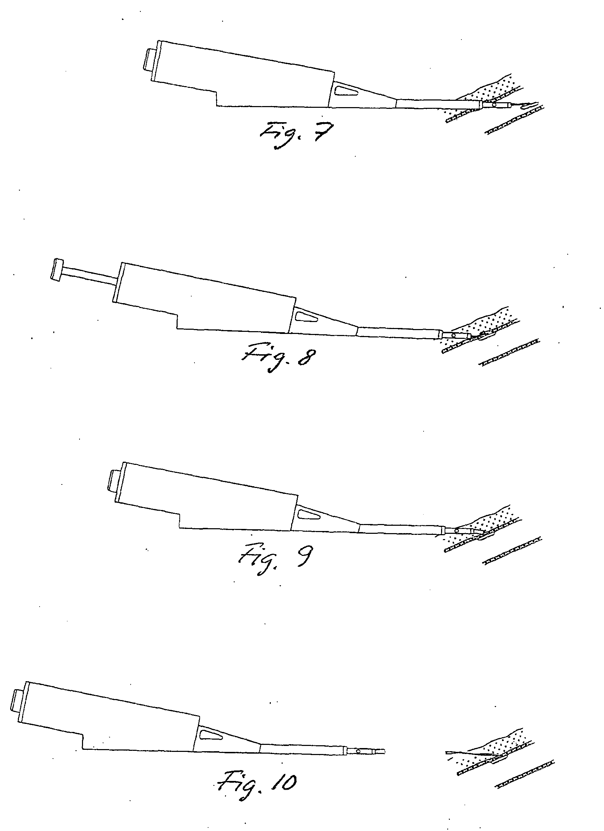

As illustrated in fig. 7, an actuator is depressed into the proximal end of the closure device housing. When the distal end of the device is positioned in the blood vessel, the guide rod is removed, and the operator pushes the actuator towards an end position provided in the housing. The actuator is operatively associated, in a first relative position, with a sliding member that carries a tamping tube and a pusher, the latter detachably carrying the inner seal by its distal end and pushing the inner seal out from the closure device to be deployed inside the vessel. This step completes a first mode of operation of the actuator mechanism.

One embodiment of the actuator mechanism, which is accommodated in the housing of the closure device 1, is diagrammatically illustrated and further explained below with reference to figs. 11-15. An important feature of the mechanism is that the locking member, which is carried behind the inner seal on a filament, cannot be pushed out from the closure device unintentionally when the actuator has been pushed into its end position. An erroneous tamping of the locking member inside the vessel is thereby prevented. The mechanism comprises a spring member generating a biasing force acting on the actuator as the actuator is pushed into the housing. When the actuator reaches its end position, a snap lock connection temporarily arrests the actuator in this end position.

In the next step of the sealing operation the housing is manually retracted, i.e. the housing is pulled in the proximal direction until the inner seal is seated over the puncture in contact with the inner surface of the vessel wall, while simultaneously the distal end of the closure device is retracted from the puncture as is illustrated in fig. 8.

During retraction of the closure device 1, the pulling force is carried by the filament which is arrested by its distal end being attached to the inner seal and by its proximal end being connected to the sliding member. The filament thus prevents the sliding member from moving in the proximal direction, causing the sliding member to be displaced relative to the actuator. By means of a cam surface provided on the sliding member, the snap connection that arrests the actuator is disengaged in response to the relative displacement between the retracting actuator and the stationary sliding member. As the snap connection is disengaged, the biasing spring pushes the actuator back to a tamping position wherein the actuator is again operatively associated with the sliding member, now in a second relative position. Furthermore, during this retraction of the housing, the proximal end of the pusher is disconnected from engagement with the sliding member, and a subsequent second forward motion of the actuator will have no effect on the pusher. In the tamping operation the actuator acts on the sliding member, the tamping tube and the locking member.

Fig. 9 illustrates the actions caused by a second forward motion of the actuator. When the actuator this second time is pushed towards its end position in the housing, the actuator pushes on the sliding member which, via the tamping tube, pushes the locking member forward and into a locking position against the outer surface of the vessel wall, as is illustrated in fig. 9. This step completes the second mode of operation of the actuator mechanism.

The filament runs through the locking member. In the tamped position, the filament secures the locking member by means of frictional engagement provided from a distal portion of the filament, said portion having an enlarged dimension or diameter.

The proximal end of the filament is attached to the sliding member through a sliding connection. In the first mode, when the actuator and sliding member are pushed forward for deployment of the inner seal, the filament is tensioned by the pusher gripping the inner seal by its distal end. In the second mode, as the actuator and sliding member are pushed forward for tamping the locking member, the pusher is inactive and the filament is arrested by a member which is stationary relative to the sliding member and which maintains a tension of the filament. At the very end of the forward motion, the proximal end of the filament is released from the sliding member by the action of the stationary member.

Here it should be noted that one person can perform the closure using one hand only throughout the whole sealing procedure.

The housing and its associated components can now be removed and disposed of, thereby leaving only the inner seal, the locking member and the filament in place, as is illustrated in fig. 10. Cutting the filament completes the sealing operation.

As will be more fully understood from the following description of the device, the present invention discloses a method for sealing a percutaneous puncture in the wall of a blood vessel, comprising the step of providing an insertion tool having an actuator which is operable in a first mode for deployment of an inner seal inside the vessel and operable in a second mode for tamping of a locking member on the outside of the vessel, wherein operation of the actuator for tamping the locking member is enabled through the step of applying a pulling force to act on a filament, connecting the inner seal and the locking member, for setting the actuator into said second operable mode.

Preferably, the step of operating the actuator for deployment of the inner seal disables operation of the actuator for tamping the locking member, until the step of applying a pulling force to act on the filament resets the actuator into said second operable mode.

### REALIZATION OF A CLOSURE DEVICE ACCORDING TO THE INVENTION

In the following description of an illustrated embodiment, "distal" refers to the left hand side and "proximal" refers to the right hand side of the drawings of figs. 11-15. Also, the expressions "top", "bottom", "horizontal" and "vertical" refers entirely to the orientation shown in the drawings, and is no indication of the actual orientation of the closure device in practise.

### MAJOR COMPONENTS OF THE CLOSURE DEVICE

With reference to fig. 11, the closure device 1 comprises as its major components: a housing 100 (outlined in dash-dot lines), and an actuator 200, a slider 300, a sleeve 400, a seal assembly 500, all supported in the housing.

Basically, the sleeve is telescopically received in the housing, the actuator is telescopically received in the sleeve, the seal assembly is operatively connected to the slider, and the slider is operatively engaged by the actuator. The slider 300 is guided for longitudinal displacement relative to the actuator 200 between first and second relative positions, in which the actuator operatively engages the slider to be brought into the movement of the actuator. The actuator 200 is guided for longitudinal movement relative to the sleeve 400, from an extended storage or idle position to a partially overlapping operable position from where the actuator is further advanced to an end position for deployment of the inner seal in the first mode, and for tamping the locking member in the second mode of operation. The sleeve 400 is accommodated in the housing 100 and guided therein for longitudinal movement, from an extended idle position to a fully inserted operative position. Advantageously, the housing, sleeve and actuator are arranged about a common longitudinal axis. The housing, the sleeve and the actuator, as well as the slider, may be of any suitable sectional profile, such as circular, or they may for example have an orthogonal section such as the illustrated embodiment of a closure device according to the invention. As an assembly, theses components provide a tool for insertion of the sealing components such as the inner seal, the filament and the locking member.

### THE HOUSING

The housing 100 is associated with an insertion tube 101, connecting to the distal end of the housing via a forward house portion 102 (as seen in the direction of insertion). Insertion tube 101 and house portion 102 may be integral parts of the housing. When inserted by its distal end through the puncture, the insertion tube 101 communicates the blood vessel with first and second passageways formed in the forward house portion 102, one of which is designed to receive a guide that controls the insertion tube during location in the blood vessel, and the other passageway designed for insertion of the inner seal and locking member upon sealing of the puncture. These passageways converge into the insertion tube 101. Advantageously, the housing is formed with indication means providing a verification that a flow communication has been established with the blood vessel, via the distal end of housing 100/insertion tube 101.

### THE SEAL ASSEMBLY

With reference to fig. 12, the seal assembly 500 comprises the inner seal 501 and the locking member 502, the inner seal being anchored in the distal end of the suture or filament 503 running through the locking member. The locking member 502 is spaced behind the inner seal, on the proximal side of an end portion 504 of the filament having increased sectional dimension, providing a frictional engagement with the locking member in its tamped (advanced) position on the filament. In a ready-to-use condition, the inner seal and locking member both lie encapsulated in the forward house portion 102 (diagrammatically illustrated in fig. 11).

The filament 503 runs through the tamping tube 505, together with the pusher 506. The filament and the pusher both reach out beyond the distal end of the tamping tube, the pusher by its distal end detachably gripping the inner seal 501, and the locking member 502 freely supported on the filament outside the distal end of the tamping tube 505. Also, the filament and the pusher both reach out from the proximal end of the tamping tube. The proximal ends of the filament 503, the pusher 506 and the tamping tube 505 are all supported in the slider 300, as will be explained below with reference also to figs. 13a-13d.

### THE SLIDER

In the illustrated embodiment, the slider 300 is an elongate four-sided body having an orthogonal section, dimensioned to be accommodated in the actuator 200 for longitudinal movement therewith, and guided in the actuator for longitudinal displacement relative thereto. In figs. 13c-13d, the slider 300 has opposed, vertical side walls 301,302 connecting a horizontal top plane 303 with a horizontal bottom plane 304. Preferably, the longitudinal margins connecting to the walls are chamfered in order to facilitate a sliding displacement free from jamming in the actuator.

The tamping tube 505 connects to the distal end of the slider: the proximal end of the tamping tube being received in a recess 305, mouthing in the distal end of the slider 300.

In one side wall 301 of the slider 300, the recess 305 opens laterally towards the exterior, the opening forming a slot 306 that connects the recess 305 with a clearance 307 through the side wall 301. On the interior of side wall 301, the slot 306 proceeds rectilinearly from the clearance 307 to the proximal end of slider 300. A corresponding slot 306' is formed on the interior of the opposite side wall 302. The slots 306,306' are dimensioned to receive and to guide the pusher 506, the proximal end 507 of the pusher being bent transversely to reach across the interior of slider 300 for a sliding engagement with the two slots 306,306'.

The filament is detachably connected to the slider through a sliding connection. The proximal end of the filament 503 is arrested in the slider by being looped around a longitudinal bar 308, cut out from the opposite side wall 302. The bar 308 extends from a distal portion of the slider, and terminates with a free end in a proximal portion of the slider. The filament runs, under a slight tension, from the bar 308 across the interior of slider 300, via the clearance 307 into the slot 306 and further through the tamping tube 505 to the inner seal which is supported in the distal end of the pusher 506.

The tension of the filament is provided by the pusher: the transverse portion in the proximal end 507 of the pusher being arrested in a seat 309 formed in a latch 310 that rises from the bottom 304 of slider 300, and the distal end of the pusher being detachably connected to the inner seal which is attached to the distal end of the filament. The length of the pusher is determined with respect to the length of the filament to provide a slight bias of the pusher, sufficient for tensioning the filament as long as the proximal end of the pusher is arrested by the latch 310, and the proximal end of the filament is looped around the bar 308.

The seat 309 provides a snap lock connection with the pusher, by the latch 310 being depressible towards the bottom of slider 300. Depression of the latch causes the pusher to be released from the seat 309 and free to slide in the slots 306,306', towards the proximal end of the slider 300.

A heel 311, facing the proximal end of slider 300, is formed in the terminal end of an arm 312 rising from the bottom of the slider. Similar to the latch 310, the arm 312 is depressible towards the bottom of slider 300. However, for reasons that will be explained further on, the arm 312 is flexible and able to return to an operative position shown in the drawings. Advantageously, the latch 310 and the arm 312 are both flexible and formed integrally with the slider, made for example of a synthetic material such as a polymer material.

The latch 310 and the arm 312 are both formed with ramp surfaces 313,314 interacting with a cam which is stationary in the actuator 200. When assembled, the cam reaches through the open top plane 303 to be received in the interior of slider 300 as will be further explained with reference to figs. 14a-e.

### THE ACTUATOR

In the illustrated embodiment of figs. 14a-e, the actuator 200 is an elongate, hollow, four-sided body having an orthogonal section, dimensioned to receive the slider 300 for longitudinal movement therewith, and guiding the slider for longitudinal displacement relative thereto. The actuator 200 is formed with opposed, vertical side walls 201,202 connecting a horizontal top plane 203 with a horizontal bottom plane 204. Preferably, the longitudinal margins connecting to the walls are chamfered in order to facilitate a sliding movement free from jamming in the sleeve 400.

With reference to figs. 14a-14c, the actuator 200 is a two-piece element hinged together along one side thereof. A snap lock engagement may be formed on the opposite side for closing the actuator body. The interior of actuator 200 is divided into a first longitudinal chamber 205 formed to receive the slider 300, and a second longitudinal chamber 206 formed to receive a compressible spring 207 (shown diagrammatically through dash-dot lines in figs. 14b, 14c). Both chambers, separated by a longitudinal partition wall 208, opens in the distal end of actuator 200. The proximal end thereof is closed, carrying a push-button 209.

Depending from the interior of top plane 203, a cam 210 reaches down into the chamber 205. In the assembled position, the cam 210 is received through the open top plane of the slider 300 to a depth wherein the cam 210 is operative to engage and depress the ramp surfaces 313 and 314 in succession, i.e. first depressing the latch 310 and then the arm 312, when the slider is displaced relative to the actuator.

In the first mode of operation for deployment of the inner seal, figs. 7 and 14d, the proximal end of the slider 300 abuts the proximal push-button end of actuator 200, the actuator thus pushing the slider forward (towards the distal end) in a first relative position between slider and actuator.

In the second mode of operation for tamping the locking member, figs. 9 and 14e, the cam 210 engages the heel 311 and the actuator thus pushing the slider in a second, advanced position relative to the actuator.

The displacement of the slider 300, from said first to said second position relative to the actuator, is caused by applying a pulling force upon retraction of the housing in order to position the inner seal over the puncture and in order to retract the distal end of the housing/insertion tube (see fig. 8). During retraction, the slider will remain stationary relative to the inner seal, connected therewith through the filament. A pulling force, applied to the actuator via the housing and the sleeve, and thus acting on/carried by the filament causes ejection of the spring biased actuator, and brings the cam 210 to engage the ramp on latch 310 which is depressed by the moving cam to release the pusher from the seat 309. Further motion of the actuator cam 210 will bring the pusher along, the transverse portion 507 of the pusher being caught by a hook 211 that is formed on the cam 210. Next, the cam 210 engages the ramp on arm 312, which is depressed to let the cam pass to the proximal side of the heel 311. Due to the flexibility of arm 312, the arm returns to its original position wherein the heel 311 projects into the path of the cam, thus arresting the slider in a second and advanced position relative to the actuator. Simultaneously, the proximal end of the pusher leaves the guiding slots 306,306' formed on the interior of the slider walls. As the pusher is released from engagement with the seat 309 and pulled backwards by the cam-and-hook formation 210,211, the distal end of the pusher is concurrently disconnected from the inner seal. When the proximal end of the pusher leaves the slots in the slider walls, the distal end of the pusher is also fully retracted into the distal end of the tamping tube.

The relative displacement between the actuator and the slider is thus initiated by the pulling force acting on the filament, and is then driven by the compressible spring as will be explained below.

### THE SLEEVE

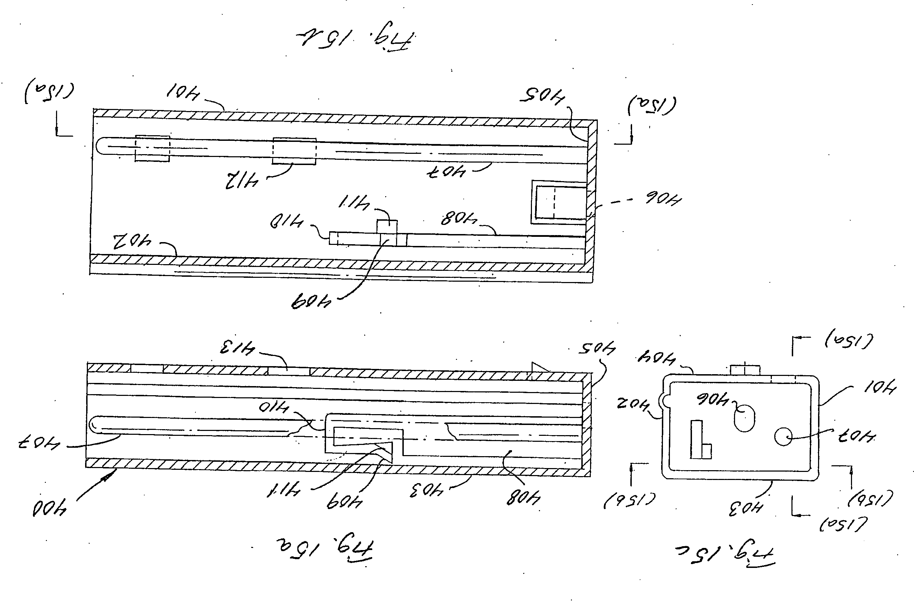

With reference to figs. 15a-c, the sleeve 400 is an elongate, hollow, four-sided body having an orthogonal section, dimensioned to receive and guide the actuator 200 for longitudinal movement relative to the sleeve. The sleeve 400 is formed with opposed, vertical side walls 401,402 connecting a horizontal top plane 403 with a horizontal bottom plane 404. Preferably, the longitudinal margins connecting to the walls are chamfered in order to facilitate a jam free movement relative to the housing 100, accommodating the sleeve 400.

The sleeve 400 has an open proximal end receiving the actuator, and the distal end of the sleeve being closed by an end wall 405. An opening 406 through the end wall communicates with the passage through the forward portion 102 of the housing 100, guiding the seal assembly into the insertion tube of the closure device.

A rod 407 projects longitudinally through the sleeve, from the end wall 405 towards the proximal end. In the assembly, the rod 407 projects into the chamber 206 of the actuator to support the spring 207, in this case a coiled spring 207, acting between the end wall 405 of the sleeve and the proximal or push-button end 209 of the actuator, and being compressed when the actuator is depressed into the sleeve.

Also projecting from the end wall 405 is a beam 408, running in parallel with the rod and aligned with the open interior of the slider 300 in the assembled position. The beam 408 runs with a clearance from the interior of top plane 403, substantially corresponding to a wall thickness in the top plane 203 of actuator 200. The beam 408 carries a latch 409 which is depressible towards the bottom plane of the sleeve. The latch 409 is flexible to engage a slot 212 (see fig. 14a), provided in the top plane 203 of the actuator when the actuator is fully depressed into the sleeve for deployment of the inner seal. The slot 212 and latch 409 provide a snap lock connection, the latch 409 being operative for retaining, temporarily as will be explained below, the actuator with its distal end abutting the end wall 405 of the sleeve. In this end position of the actuator, which terminates the first mode of operation in a definite stop from where the actuator is prevented from further movement in the distal direction, the proximal end 410 of the beam is received in the distal end of the slider 300 which is engaged by the actuator in said first relative position.

### RESET OF THE ACTUATOR

The latch 409 is associated with a ramp surface 411 which is laterally and vertically offset from the latch and aligned to be operatively engaged by a cam 315, provided in the distal end of the slider (see figs. 13a-d). Upon retraction of the insertion tool, the cam 315 on the slider (which remains stationary) acts on the ramp surface 411 to depress the latch 409, which is then disengaged from the slot 212. The actuator 200 is thus disengaged from the snap lock connection 212,409 to be ejected by action of the spring 207, the spring driving the actuator in the proximal direction relative to the sleeve and relative to the stationary slider.

The action of the spring causes the release of the pusher, and resets the actuator into a position from where the actuator is operable and enabled for tamping the locking member. The spring actuated ejection of the actuator is limited through a flexible latch 213, provided in a distal end portion of the actuator, snapping into engagement with a slot 412 provided on the sleeve. The latch 213 is formed with a ramp surface facing a distal margin of the slot, and a transverse surface engaging a proximal margin of the slot 413. From this arrested position the actuator 200 is operable to be depressed in the distal direction, but is however prevented from further movement in the proximal direction. Depression of the actuator into the sleeve will bring the slider 300 in the movement of the actuator, the cam 210 of the actuator engaging the heel 311 of the slider in the second, advanced position relative to the actuator.

### RELEASE OF THE FILAMENT

When the actuator from this position is depressed into the sleeve in the second mode of operation, the slider is advanced in a forward or distal portion of the actuator. In this movement, the beam 408 which is stationary on the sleeve, moves longitudinally through the interior of the slider. The filament which is looped around the bar 308 and crossing the interior of the slider, is then captured by the proximal end 410 of the beam and caused thereby to slide towards the proximal end of the bar 308. Accordingly, the filament remains stationary while the actuator, slider and tamping tube are advanced for slipping the locking member into frictional engagement with the distal portion of the filament. At the end of the tamping operation the filament loop has reached the proximal end of the bar, from where it is slipped off by action of the beam 408, and the filament thus being released from the slider. Obviously, all structures involved in the tamping operation are dimensioned with respect to structural lengths and lengths of movement to allow release of the filament as the locking member has reached its final position on the filament, tamped against the outside of the vessel wall. Release of the filament terminates the tamping operation, and the insertion tool can be removed from the patient.

The illustrated embodiment is one example of realization of the invention. Modifications to the detailed structure and design of components are possible without changing the basic solution, defined by the claims.

Instead of one integral actuator, e.g., a first actuator may be adapted for deployment of the inner seal and a second actuator being adapted for tamping the locking member. In such case the slider would be arranged to be disengaged from the first actuator and brought into engagement with the second actuator in response to a pulling force acting through the filament, while simultaneously the second actuator is set into an operable condition. Also, instead of being arranged about a common longitudinal axis as illustrated, the actuator, sleeve and slider may be of any conceivable shape as long as it is ensured that the first mode of operation terminates in a definite stop from where the tamping tube and locking member are prevented from further movement in the distal direction until the second mode of operation is enabled in response to a pulling force being applied to act on the filament.

A central feature of the new closure device is, accordingly, that an actuator in a first mode is operable for deployment of an inner seal, and is then reset into a second mode wherein the actuator is operable for tamping a locking member. Reset of the actuator is accomplished by applying a pulling force to act on the filament that connects the inner seal and the locking member. Thus, deployment of the inner seal, enabling of a second operable mode and tamping of the locking member may all be performed in a one-hand operation. Also, an erroneous positioning of the locking member inside the blood vessel is prevented by the actuator preferably being disabled until a pulling force acting on the filament causes the actuator to be reset into an operable condition. A proper closure of the percutaneous puncture and ease of handling are thus both conceivably enhanced through a closure device as disclosed by the present invention.

## Claims

1. A closure device by which a percutaneous puncture in the wall of a blood vessel is closable by an inner sealing member (501) adapted to be deployed inside the blood vessel and an outer locking member (502) adapted to be tamped on the outside of the blood vessel, the closure device comprising
- a housing (100) supporting the sealing and locking members (501; 502);
- an actuator mechanism (300, 400) movably received in the housing;
- an actuator (200) movably received in the housing (100), the actuator (200) being accessible from outside the housing while in operative engagement with the actuator mechanism (300, 400);
- a filament (503) connecting the inner sealing member (501) with the actuator mechanism (300, 400), wherein
- the actuator mechanism (300, 400) in a first mode of operation is effective for deployment of the inner sealing member (501), and
- the actuator mechanism (300, 400) in a second mode of operation is effective for tamping the outer locking member (502), **characterized in that** the actuator (200) is manually operable for controlling the actuator mechanism (300, 400) in both said first and second modes of operation, and **in that** the actuator (200) is set into the second mode of operation in response to a pulling force applied to the actuator mechanism (300, 400) via the filament (503).

2. The closure device of claim 1, wherein the actuator (200), in response to the pulling force applied via the filament (503), is ejected into said second mode through the action of a spring member (207).

3. The closure device of claim 1 or 2, wherein the housing (100) is arranged to be positioned by its distal end for guidance of a seal assembly, the seal assembly comprising
- the inner sealing member (501), attached to the distal end of the filament (503);
- the outer locking member (502), movably carried on the filament and spaced from the inner sealing member;
- a pusher (506), guided in the housing, the distal end of the pusher detachably connected to the inner sealing member;
- a tamping tube (505), guided in the housing, the filament (503) running through the tamping tube and carrying the outer locking member and the inner sealing member outside the distal end of the tamping tube,
wherein the actuator mechanism (300,400) is controllable by the actuator (200) in an operable first mode to drive the pusher for deployment of the inner sealing member (501), whereupon the actuator mechanism is adapted to disable the actuator until a pulling force acting on the filament causes the actuator to be reset into an operable second mode to drive the tamping tube for tamping the outer locking member (502).

4. The closure device of claim 3, wherein
- a sleeve (400) is telescopically received in the housing (100);
- the actuator (200) is telescopically received in the sleeve;
- a slider (300) is accommodated in the actuator (200);
- the seal assembly is operatively connected to the slider via the filament (503), and
wherein the slider (300) is engaged by the actuator (200) in a first relative position to be moved by the actuator for deployment of the inner sealing member (501), and in result of a pulling force acting on the filament (503) displaceable relative to the actuator into a second relative position wherein the slider is engaged by the actuator to be moved by the actuator for tamping the outer locking member (502).

5. The closure device of claim 4, wherein the slider (300) is displaced from the first relative position to the second relative position in response to a pulling force being applied to the closure device and acting through the filament (503) which is arrested by its distal end being attached to the inner seal (501) and by its proximal end being connected to the slider.

6. The closure device of claim 5, wherein the actuator (200) is temporarily arrested in the sleeve (400) and disabled in the second mode of operation, to be released by action of the slider (300) as the slider is displaced from said first to said second position relative to the actuator.

7. The closure device of claim 6, wherein a cam formation (315) on the slider is arranged to disengage a snap lock connection (212, 409) between the actuator (200) and the sleeve (400), and a compressible spring (207), acting between the sleeve and the actuator, is effective for ejecting the actuator into said second operable mode.

8. The closure device of claim 7, wherein the pusher (506) is released from the slider (300) in response to the actuator (200) being reset into said second operable mode.

9. The closure device of claim 8, wherein a cam-and-hook formation (210,211) on the actuator (200) is arranged to disengage a snap lock connection (309,507) between the slider (300) and the pusher (506), and further to retract the pusher by the displacement of the slider relative to the actuator.

10. The closure device of claim 5, wherein the proximal end of the filament (503) is connected to the slider (300) by a sliding connection (308), and captured by a stationary member (411) on the sleeve (400) to be released from the slider through the relative movement of the stationary member as the slider moves with the actuator (200) in the second mode of operation.

11. The closure device of any previous claim, wherein an insertion tool (1) comprises a housing (100), the distal end of which is associated with an insertion tube (101), and an interconnecting, forward portion (102) of the housing has separate passageways for the seal assembly (500) and for a guiding member, respectively, said passageways converging into the insertion tube.

12. The closure device of claim 11, wherein the housing, the forward portion and the insertion tube are integrally formed.

## Patentansprüche

1. Verschlussvorrichtung, mit der eine perkutane Punktion in der Wand eines Blutgefäßes durch ein inneres Dichtungselement (501), das in dem Blutgefäß abgelegt werden kann, und ein äußeres Arretierelement (502), das auf der Außenseite des Blutgefäßes festgestampft werden kann, verschlossen werden kann, wobei die Verschlussvorrichtung
- ein Gehäuse (100), das die Dichtungs- und Arretierelemente (501; 502) stützt;
- einen Betätigungsmechanismus (300, 400), der beweglich im Gehäuse aufgenommen ist;
- ein Betätigungsglied (200), das beweglich im Gehäuse (100) aufgenommen ist, wobei das Betätigungsglied (200) von der Außenseite des Gehäuses her zugänglich ist, während es sich in Betriebseingriff mit dem Betätigungsmechanismus (300, 400) befindet);
- einen Faden (503), der das innere Dichtungselement (501) mit dem Betätigungsmechanismus (300, 400) verbindet, worin
- der Betätigungsmechanismus (300, 400) in einem ersten Betriebsmodus zur Ablage des inneren Dichtungselements (501) dient und
- der Betätigungsmechanismus (300, 400) in einem zweiten Betriebsmodus zum Aufstampfen des äußeren Arretierelements (502) dient, **dadurch gekennzeichnet, dass** das Betätigungsglied (200) zur Steuerung des Betätigungsmechanismus (300, 400) in den ersten und zweiten Betriebsmodus manuell bedient werden kann und dass das Betätigungsglied (200) als Reaktion auf eine Zugkraft auf den Betätigungsmechanismus (300, 400) durch den Faden (503) in den zweiten Betriebsmodus gesetzt wird.

2. Verschlussvorrichtung nach Anspruch 1, worin das Betätigungsglied (200) als Reaktion auf die Zugkraft, die durch den Faden (503) aufgebracht wird, durch die Wirkung eines Federelements (207) in den zweiten Modus abgeworfen wird.

3. Verschlussvorrichtung nach Anspruch 1 oder 2, worin das Gehäuse (100) mit seinem distalen Ende zur Führung einer Dichtungsanordnung angeordnet werden kann, wobei die Dichtungsanordnung
- das innere Dichtungselement (501), das am distalen Ende des Fadens (503) befestigt ist;
- das äußere Arretierelement (502), das beweglich auf dem Faden getragen und im Abstand vom inneren Dichtungselement gehalten wird;
- einen Schieber (506), der im Gehäuse geführt wird, wobei das distale Ende des Schiebers lösbar mit dem inneren Dichtungselement verbunden ist;
- ein Stampfrohr (505), das im Gehäuse geführt wird, wobei der Faden (503) durch das Stampfrohr läuft und das äußere Arretierelement und das innere Dichtungselement außerhalb des distalen Endes des Stampfrohrs trägt, umfasst,
worin der Betätigungsmechanismus (300, 400) durch das Betätigungsglied (200) in einem ersten Betriebsmodus so gesteuert werden kann, dass es den Schieber zur Ablage des inneren Dichtungselements (501) antreibt, woraufhin der Betätigungsmechanismus das Betätigungsglied deaktivieren kann, bis eine Zugkraft auf den Faden dazu führt, dass das Betätigungsglied wieder in einen betriebsfähigen zweiten Modus gesetzt wird, um das Stampfrohr zum Stampfen des äußeren Arretierelements (502) anzutreiben.

4. Verschlussvorrichtung nach Anspruch 3, worin
- eine Hülse (400) teleskopartig im Gehäuse (100) aufgenommen ist;
- das Betätigungsglied (200) teleskopartig in der Hülse aufgenommen ist;
- ein Gleitstück (300) im Betätigungsglied (200) untergebracht ist;
- die Dichtungsanordnung über den Faden (503) mit dem Gleitstück in Wirkverbindung steht und worin das Gleitstück (300) vom Betätigungsglied (200) in einer Relativstellung ergriffen wird, um vom Betätigungsglied zur Ablage des inneren Dichtungselements (501) bewegt zu werden und durch eine Zugkraft auf den Faden (503) relativ zum Betätigungsglied in eine zweite Relativstellung verschoben werden kann, in der das Gleitstück vom Betätigungsglied ergriffen wird, um vom Betätigungsglied zum Stampfen des äußeren Arretierelements (502) bewegt zu werden.

5. Verschlussvorrichtung nach Anspruch 4, worin das Gleitstück (300) als Reaktion auf eine Zugkraft auf die Verschlussvorrichtung durch den Faden (503), der arretiert wird, indem sein distales Ende an der inneren Dichtung (501) befestigt ist und indem sein proximales Ende mit dem Gleitstück verbunden ist, aus der ersten Relativstellung in die zweite Relativstellung verschoben wird.

6. Verschlussvorrichtung nach Anspruch 5, worin das Betätigungsglied (200) temporär in der Hülse (400) arretiert wird und im zweiten Betriebsmodus deaktiviert wird, um durch die Wirkung des Gleitstücks (300) freigesetzt zu werden, wenn das Gleitstück aus der ersten in die zweite Stellung relativ zum Betätigungsglied verschoben wird.

7. Verschlussvorrichtung nach Anspruch 6, worin eine Nockenformation (315) auf dem Gleitstück eine Schnappverschlussverbindung (212, 409) zwischen dem Betätigungsglied (200) und der Hülse (400) zu lösen und eine Druckfeder (207), die zwischen der Hülse und dem Betätigungsglied wirkt, das Betätigungsglied in den zweiten Betriebsmodus abwerfen kann.

8. Verschlussvorrichtung nach Anspruch 7, worin der Schieber (506) als Reaktion auf das Zurücksetzen des Betätigungsglieds (200) in den zweiten Betriebsmodus vom Gleitstück (300) gelöst wird.

9. Verschlussvorrichtung nach Anspruch 8, worin eine Nocken-und-Haken-Formation (210, 211) auf dem Betätigungsglied (200) eine Schnappverschlussverbindung (309, 507) zwischen dem Gleitstück (300) und dem Schieber (506) lösen und den Schieber durch Verschiebung des Gleitstücks relativ zum Betätigungsglied weiter zurückziehen kann.

10. Verschlussvorrichtung nach Anspruch 5, worin das proximale Ende des Fadens (503) über eine Gleitverbindung (308) mit dem Gleitstück (300) verbunden ist und von einem feststehenden Element (411) auf der Hülse (400) festgehalten wird, um durch die Relativbewegung des feststehenden Elements freigesetzt zu werden, wenn das Gleitstück sich mit dem Betätigungsglied (200) in den zweiten Betriebsmodus bewegt.

11. Verschlussvorrichtung nach einem der vorhergehenden Ansprüche, worin ein Einführinstrument (1) ein Gehäuse (100) umfasst, dessen distales Ende mit einem Einführrohr (101) in Verbindung steht, und wobei ein vorderer Verbindungsabschnitt (102) des Gehäuses separate Durchgänge für die Dichtungsanordnung (500) und für ein Führungselement aufweist, wobei die Durchgänge in das Einführrohr konvergieren.

12. Verschlussvorrichtung nach Anspruch 11, worin das Gehäuse, der vordere Abschnitt und das Einführrohr einstückig geformt sind.

## Revendications

1. Dispositif de fermeture qui permet de fermer une perforation percutanée dans la paroi d'un vaisseau sanguin à l'aide d'un organe d'étanchéité interne (501) conçu pour être déployé à l'intérieur du vaisseau sanguin et d'un organe de verrouillage externe (502) conçu pour être tassé sur l'extérieur du vaisseau sanguin, le dispositif de fermeture comportant
- un boîtier (100) supportant les organes d'étanchéité et de verrouillage (501 ; 502) ;
- un mécanisme actionneur (300, 400) reçu de manière mobile dans le boîtier ;
- un actionneur (200) reçu de manière mobile dans le boîtier (100), l'actionneur (200) étant accessible depuis l'extérieur du boîtier quand il est en prise fonctionnellement avec le mécanisme actionneur (300, 400), dans lequel
- un filament (503) reliant l'organe d'étanchéité interne (501) au mécanisme actionneur (300, 400), dans lequel
- le mécanisme actionneur (300, 400) dans un premier mode de fonctionnement sert à déployer l'organe d'étanchéité interne (501), et
- le mécanisme actionneur (300, 400) dans un deuxième mode de fonctionnement sert à tasser l'organe de verrouillage externe (502), **caractérisé en ce que** l'actionneur (200) est utilisable à la main pour contrôler le mécanisme actionneur (300, 400) à la fois dans lesdits premier et deuxième modes de fonctionnement, et **en ce que** l'actionneur (200) est réglé dans le deuxième mode de fonctionnement en réponse à une force de tirage appliquée sur le mécanisme actionneur (300, 400) par l'intermédiaire du filament (503).

2. Dispositif de fermeture selon la revendication 1, dans lequel l'actionneur (200), en réponse à la force de tirage appliquée par l'intermédiaire du filament (503), est éjecté dans ledit deuxième mode au travers de l'action d'un organe à ressort (207).

3. Dispositif de fermeture selon la revendication 1 ou 2, dans lequel le boîtier (100) est disposé de façon à être positionné par son extrémité distale pour guider un ensemble d'étanchéité, l'ensemble d'étanchéité comportant
- l'organe d'étanchéité interne (501), fixé à l'extrémité distale du filament (503) ;
- l'organe de verrouillage externe (502), porté de manière mobile sur le filament et à distance de l'organe d'étanchéité interne ;
- un poussoir (506), guidé dans le boîtier, l'extrémité distale du poussoir étant reliée de manière détachable à l'organe d'étanchéité interne ;
- un tube de tassement (505), guidé dans le boîtier, le filament (503) passant à travers le tube de tassement et portant l'organe de verrouillage externe et l'organe d'étanchéité interne à l'extérieur de l'extrémité distale du tube de tassement,
dans lequel le mécanisme actionneur (300, 400) peut être contrôlé par l'actionneur (200) dans un premier mode d'utilisation pour entraîner le poussoir de façon à déployer l'organe d'étanchéité interne (501), le mécanisme actionneur étant conçu pour désactiver l'actionneur jusqu'à ce qu'une force de tirage agissant sur le filament provoque la remise à l'état initial de l'actionneur dans un deuxième mode d'utilisation pour entraîner le tube de tassement de façon à tasser l'organe de verrouillage externe (502).

4. Dispositif de fermeture selon la revendication 3, dans lequel
- un manchon (400) est reçu de manière télescopique dans le boîtier (100) ;
- l'actionneur (200) est reçu de manière télescopique dans le manchon ;
- une pièce coulissante (300) est logée dans l'actionneur (200) ;
- l'ensemble d'étanchéité est relié fonctionnellement à la pièce coulissante par l'intermédiaire du filament (503), et la pièce coulissante (300) est en prise avec l'actionneur (200) dans une première position relative pour être déplacée par l'actionneur afin de déployer l'organe d'étanchéité interne (501), et à cause d'une force de tirage agissant sur le filament (503) elle peut être déplacée par rapport à l'actionneur dans une deuxième position relative, la pièce coulissante étant en prise avec l'actionneur pour être déplacée par l'actionneur de façon à tasser l'organe de verrouillage externe (502).

5. Dispositif de fermeture selon la revendication 4, dans lequel la pièce coulissante (300) est déplacée de la première position relative à la deuxième position relative en réponse à une force de tirage appliquée sur le dispositif de fermeture et agissant à travers le filament (503) qui est arrêté par son extrémité distale fixée à l'étanchéité interne (501) et par son extrémité proximale reliée à la pièce coulissante.

6. Dispositif de fermeture selon la revendication 5, dans lequel l'actionneur (200) est temporairement arrêté dans le manchon (400) et désactivé dans le deuxième mode de fonctionnement, pour être libéré par l'action de la pièce coulissante (300) au fur et à mesure que celle-ci est déplacée de ladite première à ladite deuxième position relativement à l'actionneur.

7. Dispositif de fermeture selon la revendication 6, dans lequel une formation de came (315) sur la pièce coulissante est disposée de façon à désengager un raccord à verrouillage par encliquetage (212, 409) entre l'actionneur (200) et le manchon (400), et un ressort compressible (207), agissant entre le manchon et l'actionneur, sert à éjecter l'actionneur dans ledit deuxième mode d'utilisation.

8. Dispositif de fermeture selon la revendication 7, dans lequel le poussoir (506) est libéré de la pièce coulissante (300) en réponse à la remise à l'état initial de l'actionneur (200) dans ledit deuxième mode d'utilisation.

9. Dispositif de fermeture selon la revendication 8, dans lequel une formation de came à crochet (210, 211) sur l'actionneur (200) est disposée pour désengager un raccord à verrouillage par encliquetage (309, 507) entre la pièce coulissante (300) et le poussoir (506), et en outre pour rétracter le poussoir en déplaçant la pièce coulissante relativement à l'actionneur.

10. Dispositif de fermeture selon la revendication 5, dans lequel l'extrémité proximale du filament (503) est reliée à la pièce coulissante (300) par un raccord coulissant (308), et retenue par un organe stationnaire (411) sur le manchon (400) pour être libérée de la pièce coulissante au travers du mouvement relatif de l'organe stationnaire au fur et à mesure que la pièce coulissante se déplace avec l'actionneur (200) dans le deuxième mode de fonctionnement.

11. Dispositif de fermeture selon l'une quelconque des revendications précédentes, dans lequel un outil d'insertion (1) comporte un boîtier (100), l'extrémité distale de celui-ci étant associée à un tube d'insertion (101), et une portion avant (102) d'interconnexion du boîtier présente des passages séparés respectivement pour l'ensemble d'étanchéité (500) et pour un organe de guidage, lesdits passages convergeant dans le tube d'insertion.

12. Dispositif de fermeture selon la revendication 11, dans lequel le boîtier, la portion avant et le tube d'insertion sont formés d'un seul tenant.
